(19) Europäisches Patentamt
European Patent Office
Office européen des brevets

(11) Publication number: **0 359 004 A1**

(12) **EUROPEAN PATENT APPLICATION**

(21) Application number: 89115687.9

(22) Date of filing: 25.08.89

(51) Int. Cl.[5]: A61K 31/55 , A61K 47/00 , A61L 15/16

(30) Priority: 05.09.88 JP 222081/88

(43) Date of publication of application: 21.03.90 Bulletin 90/12

(84) Designated Contracting States: AT BE CH DE FR GB IT LI LU NL SE

(71) Applicant: TAKEDA CHEMICAL INDUSTRIES, LTD.
3-6, Doshomachi 2-chome Chuo-ku
Osaka 541(JP)

(72) Inventor: Nonomura, Muneo
4-204, 6-Satsukigaoka-nishi
Suita Osaka 565(JP)
Inventor: Yamada, Masayuki
11-6, Daiwanishi 2-chome
Kawanishi Hyogo 666-01(JP)
Inventor: Nishikawa, Kohei
5-19, Oharano-kamisatotorimicho
Nishikyo-ku Kyoto 610-11(JP)

(74) Representative: Casalonga, Axel et al
BUREAU D.A. CASALONGA - JOSSE
Morassistrasse 8
D-8000 München 5(DE)

(54) **Transdermal therapeutic composition.**

(57) Antihypertensive agent, (R)-3-[(S)-1-carboxy-5-(4-piperidyl)pentyl]amino-4-oxo-2,3,4,5-tetrahydro-1,5-benzothiazepine-5-acetic acid, is absorbed through the skin in a sufficient amount and sustainedly with little dermal irritation when applied as a transdermal therapeutic composition containing (i) the antihypertensive agent, (ii) an inorganic base, (iii) at least one member selected from the group consisting of an aliphatic carboxylic acid, a lower alcohol ester thereof and an aliphatic alcohol, and (iv) an alkanepolyol.

EP 0 359 004 A1

## Transdermal Therapeutic Composition

This invention relates to a transdermal therapeutic composition which contains (i) (R)-3-[(S)-1-carboxy-5-(4-piperidyl)pentyl]amino-4-oxo-2,3,4,5-tetrahydro-1,5-benzothiazepine-5-acetic acid which has angiotensin converting enzyme inhibitory activity and is useful as a prophylactic and therapeutic agent for hypertension, namely a compound of the formula

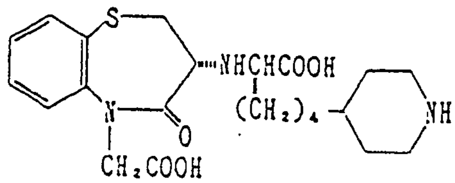

( I )

[hereinafter referred to briefly as compound (I)], (ii) an inorganic base, (iii) at least one member selected from the group consisting of an aliphatic carboxylic acid containing 6 to 20 carbon atoms, a lower alcohol ester thereof and an aliphatic alcohol containing 6 to 20 carbon atoms, and (iv) an alkanepolyol.

Recently, there has been a great interest in a transdermal therapeutic system (TTS) which is a system designed to deliver a drug through the skin to implement the intended systemic effect, and several preparations have already been developed. Thus, nitroglycerol and isosorbide dinitrate which are antianginal drugs, clonidine which is an antihypertensive agent, and scopolamine for the prevention of motion sickness have been available in TTS preparations. The first and most important consideration in TTS design is to insure absorption of a therapeutically effective amount of the drug through the skin. The second important consideration is to insure duration of the drug action, as designed, by making the most of the characteristics of transdermal absorption. Still another important requirement is that the designed system causes no side effect, such as skin irritation, on application.

Since the skin is inherently covered with a horny layer for protecting the body from penetration of foreign substances from the environment, it is generally impossible to deliver the therapeutically useful amount of a drug into the body by mere application of the drug to the skin surface. Furthermore, the skin undergoes protective reactions against the attack of extraneous foreign substances, with the result that when an attempt is made to enhance absorption of a drug with the aid of an absorption enhancer, the problem of skin irritation is usually encountered, thus frustrating the attempt to commercially implement the idea.

As a method for enhancing the transdermal absorption of drugs, the use of absorption enhancers is known and a number of transdermal absorption enhancers, including Azone® and 2-pyrrolidone, have been reported. However, all transdermal absorption enhancers are not always effective for all kinds of drugs and, for that matter, in all formulations. Rather, a given absorption enhancer is usually effective only in limited circumstances which depend on the physical properties of each drug and of each formulation. This means that one has to choose a suitable transdermal absorption enhancer for the particular circumstances at hand. Much study has also been undertaken to find methods for reducing the skin irritation potential of TTS's containing transdermal absorption enhancers but there has been little information on effective methods.

The inventors of this invention conducted an intensive study of the promotion of transdermal absorption of compound (I), sustained absorption, and alleviation of dermal irritability in application. As a result, it was found that the above-mentioned formulation results in enhanced absorption and duration of action of compound (I) with a reduced dermal irritation potential, and the present invention has been completed.

This invention provides, as mentioned hereinbefore, a transdermal therapeutic composition which contains (i) compound (I) [component (i)], (ii) an inorganic base [component (ii)], at least one member selected from the group consisting of an aliphatic carboxylic acid containing 6 to 20 carbon atoms, a lower alcohol ester thereof and an aliphatic alcohol containing 6 to 20 carbon atoms [component (iii)] and (iv) an alkanepolyol [component (iv)].

Compound (1) has for some time been known as a prophylactic and therapeutic agent for hypertension and can, for example, be synthesized by the process described in unexamined JP-A-231 668/1985 and EP-A- 156 455.

The inorganic base includes, among others, alkali metal hydroxides (for example, sodium hydroxide, potassium hydroxide), alkali metal carbonates (for example, sodium carbonate, potassium carbonate), and

alkali metal hydrogen carbonates (for example, sodium hydrogen carbonate, potassium hydrogen carbonate).

The aliphatic carboxylic acid containing 6 to 20 carbon atoms includes, among others, saturated or unsaturated aliphatic monocarboxylic acids and dicarboxylic acids, such as caproic acid, caprylic acid, capric acid, lauric acid, myristic acid, palmitic acid, stearic acid, arachic acid, decenoic acid, linderic acid, oleic acid, linoleic acid, linolenic acid, arachidonic acid, and sebacic acid.

The lower alcohol ester of aliphatic carboxylic acid containing 6 to 20 carbon atoms includes esters of the above-mentioned aliphatic carboxylic acids with lower alcohols containing 1 to about 5 carbon atoms (for example, methanol, ethanol, propanol, 2-propanol, butanol, pentanol). The lower alcohol ester of aliphatic dicarboxylic acid includes the mono- or diesters formed as one or both of the available carboxyl groups are esterified. As examples of the lower alcohol ester of $C_{6-20}$ aliphatic carboxylic acid, there may be mentioned diethyl sebacate, isopropyl myristate and so on.

The aliphatic alcohol containing 6 to 20 carbon atoms includes saturated and unsaturated aliphatic alcohols such as caproyl alcohol, caprylyl alcohol, capryl alcohol, lauryl alcohol, myristyl alcohol, cetyl alcohol, stearyl alcohol, oleyl alcohol, linoleyl alcohol, and linolenyl alcohol.

Preferred, among the aliphatic carboxylic acids lower alcohol esters thereof and aliphatic alcohols, is an ester of an aliphatic monocarboxylic acid with a lower ($C_{1-5}$) alcohol. The most desirable is isopropyl myristate.

The alkanepolyol includes, among others, lower alkanediols containing about 2 to 5 carbon atoms, such as ethylene glycol (1,2-ethanediol), propylene glycol (1,2-propanediol, 1,3-propanediol, 1,2-butanediol, 1,3-butanediol, 1,4-butanediol, 2,3-butanediol, and 1,5-pentanediol, and lower alkanetriols containing about 2 to 5 carbon atoms, such as glycerol. Particularly preferred are propylene glycol, 1,3-butanediol and glycerol.

The proportion of compound (I) in the transdermal therapeutic composition of this invention is virtually optional but preferably in the range of about 0.1 to 20 % (W/W). The proportion of the inorganic base in the pharmaceutical composition may generally range from about 0.02 to 5% (W/W), depending on its kind, and should be such that the pH of the composition will be about 6 to 9. When an alkali metal hydroxide or an alkali metal hydrogen carbonate is used as the inorganic base, its proportion is preferably about 0.8 to 1.2 mole per mole of compound (I) and when an alkali metal carbonate is used, its proportion is preferably about 0.4 to 0.6 mole per mole of compound (I).

The aliphatic carboxylic acid or the aliphatic alcohol, which is used as component (iii), is used in a proportion of, preferably, about 0.5 to 10% (W/W) and, for still better results, about 0.5 to 5% (W/W) based on the total weight of the composition. The lower alcohol ester of an aliphatic carboxylic acid, which may also be used as component (iii), is used in a proportion of, preferably, about 1 to 50% (W/W} and, for still better results, about 5 to 30% (W/W) on the same basis. When two or more kinds of aliphatic carboxylic acids, lower alcohol esters of aliphatic carboxylic acids or aliphatic alcohols are used, the total amount of the aliphatic carboxylic acids is preferably about 0.5 to 10% (W/W) and, for still better results, about 0.5 to 5% (W/W), the total amount of the lower alcohol esters of aliphatic carboxylic acids is preferably about l to 50% (W/W) and, for still better results, about 5 to 30% (W/W), and the total amount of the aliphatic alcohols is preferably about 0.5 to 10% (W/W) and, for still better results, about 0.5 to 5% (W/W).

The proportion of the alkanepolyol in this transdermal therapeutic composition is preferably about 1 to 50 % (W/W) and, for still better results, about 1 to 30 % (W/W) on the total composition.

For insuring more uniform blending of the components of this transdermal therapeutic composition, a nonionic surfactant is preferably incorporated. The proportion of the nonionic surfactant is preferably about 0.1 to 10 % (W/W) and, for still better results, about 0.5 to 5 % (W/W) based on the total composition.

The nonionic surfactant includes, among others, polyoxyethylene sorbitan fatty acid esters (for example, polyoxyethylene sorbitan monooleate, polyoxethylene sorbitan monostearate, polyoxyethylene sorbitan monopalmitate, polyoxyethylene sorbitan monolaurate, etc.), polyoxyethylene sorbitol fatty acid esters (for example, polyoxyethylene sorbitol monolaurate etc.), polyoxyethylene fatty acid esters (for example, polyoxyethylene stearate etc.), polyoxyethylene higher alcohol ethers (for example, polyoxyethylene lauryl alcohol, polyoxyethylene oleyl alcohol, etc.), polyoxyethylene alkylaryl ethers (for example, polyoxyethylene nonylphenol etc.), polyoxyethylene castor oil derivatives (for example, polyoxyethylene hydrogenated castor oil derivatives such as HCO-50, and HCO-60, etc.), polyoxyethylene lanolin derivatives, polyoxyethylene lanolin alcohol derivatives, and block polymer nonionic surfactants (for example, Pluronic, L-62, L-64, F-68, etc.).

The transdermal therapeutic composition of this invention is provided in such dosage forms as patch, cataplasma, ointment (inclusive of cream), hard ointment, tape, suppository, lotion, solution, suspension, emulsion, and aerosol mist. The ointment (inclusive of cream), suppository, lotion, solution, suspension, emulsion and aerosol can be manufactured by formulating the above-mentioned components (i), (ii), (iii) and

(iv), and if necessary the nonionic surfactant, with a solvent, suspending agent, emulsifier, propellant, ointment base, suppository base, or the like, which are well known in pharmaceutical industry. If necessary, a preservative (for example, ethyl p-hydroxybenzoate, benzalkonium chloride), antiphlogistic agent, etc. can be further incorporated. The patch, cataplasma, hard ointment and tape can be manufactured by mixing the above-mentioned components (i), (ii), (iii) and (iv), and if necessary the nonionic surfactant, with a base which is well known in pharmaceutical industry and, if necessary, after addition of a preservative, an antiphlogistic agent, etc., subjecting the mixture to absorption into, or adhesion to, an appropriate support material. The support material may be a high polymer film, a web of woven or nonwoven fabric, a sheet of paper or the like. The adhesive agent to be used in the manufacture of the patch, cataplasma or tape includes, among others, polyalkyl vinyl ether, polyalkyl acrylate, polyisobutylene, natural rubber and synthetic adhesives. For assuring suitable plasticity and tackiness, animal oil (for example, squalene, squalane, etc.) or vegetable oil (for example, olive oil, jojoba oil, etc.), petrolatum, lanolin, etc. may be added.

In the manufacture of the ointment, hard ointment, suppository, tape, patch and cataplasma, there may be incorporated substances for modulating the transdermal absorption of the drug, such as lecithin and other phospholipids, solid paraffin, bees-wax, carnauba wax, hydrogenated castor oil, lanolin, petrolatum, polyvinyl alcohol, polyvinylpyrrolidone, polyethylene glycol, glycerol fatty acid ester, cholesterol, Carbopol, carboxymethylcellulose, carboxyethylcellulose, silicone resin, and lower alcohol (for example, ethanol, isopropyl alcohol, etc.).

The solvent includes, among others, water, ethanol, and glycerol. The suspending agent includes, among others, gum arabic, carboxymethylcellulose, methylcellulose, and sodium alginate. The aerosol propellant includes, among others, non-combustible liquefied gases (for example, freon 11, freon 12, freon 113, etc.). The ointment base includes, among others, petrolatum, solid paraffin, vegetable oil, animal oil, mineral oil, lanolin, waxes, and macrogols. The hard ointment base includes, among others, bees-wax, paraffin, macrogols, and glycerol fatty acid esters. The suppository base includes, among others, cacao butter, wool fat, macrogols, Witepsol, and glycerogelatin.

When the transdermal therapeutic composition of this invention is applied to the human skin for the treatment of, for example, hypertension in an adult human, about 1 to 200 mg, preferably about 5 to 30 mg, of compound (I) is used in a single dosage form and applied once in 1 to 7 days, preferably once a day (by sticking, coating, spraying or insertion into the rectum), although the proper dosage schedule depends on the condition of the patient and other factors.

The mixing of the respective components and the manufacture of a transdermal therapeutic composition can be performed by the per se known procedures such as those described in the Japanese Pharmacopeia. Preferably, components (i) and (ii) are first dissolved in water and then the solution is blended with the other components.

The preferred dosage forms for the transdermal therapeutic composition of this invention are patches prepared using cream, gel ointment, lotion, emulsion, or hydrous gel such as polyvinyl alcohol gel.

When formulated as a transdermal therapeutic composition according to this invention, compound (I) is absorbed in a sufficient amount and sustainedly. Furthermore, dermal irritation is alleviated. Therefore, the transdermal therapeutic composition of this invention can be applied to the skin of mammalian animals (for example, human, monkey, dog, cat, rat, etc.) as a prophylactic and therapeutic agent for hypertension.

The following examples and reference example are intended to illustrate the invention in further detail and should not be construed as limiting the scope of the invention.

## Example 1

[Preparation of a transdermal therapeutic composition]

In 40 g of distilled water is dispersed 4 g of compound (I) followed by addition of 0.48 g of anhydrous sodium carbonate and the mixture is heated (at 80 - 100 °C) to dissolve compound (I). This solution is cooled to room temperature and, then, 8 g of polyvinyl alcohol (completely saponified; degree of polymerization: ca. 2,000) is added and dissolved with heating and stirring. Thereafter, 20 g of propylene glycol is added and, after mixing, 3 g of Tween 80 and 20 g of isopropyl myristate are added with stirring to give an emulsion. Finally the emulsion is diluted with distilled water to make 100 g, stirred well to homogenize, and cast onto a silicone rubber sheet provided with a recess measuring 3 $cm^2$ x 1 mm . The resulting preparation is frozen and, then, thawed to give a polyvinyl alcohol gel-type transdermal therapeutic

composition (a cataplasma).

[Transdermal absorption test]

Using 9-week-old male SD rats, the transdermal therapeutic composition (12 mg as compound (I)/rat) was applied to the clipped area (3 $cm^2$) of the abdominal skin and the area of exposure was covered by the occluded dressing method. The transdermal absorption effect was evaluated by monitoring the percent inhibition of angiotensin converting enzyme (ACE) activity. Angiotensin I was administered in an intravenous dose of 300 ng/kg (rats). The degree of sustained absorption was evaluated using the duration in hours of maintenance of 70% or greater inhibition of angiotensin converting enzyme activity as an indicator.

Dermal irritation test

The transdermal therapeutic composition was applied to the clipped skin of the back of male albino rabbits (weighing 2-3 kg) and the area of exposure was covered by occlusive dressing. The cataplasma was peeled off 24 hours after the application, and the 24-hour skin irritation effect was macroscopically observed and scored. The following Draize scale was used to calculate the dermal irritation score.

| Erythema & eschar formation | | Edema | |
|---|---|---|---|
| 0 | No erythema | 0 | No edema |
| 1 | Very slight erythema | 1 | Very slight edema |
| 2 | Well-defined erythema | 2 | Slight edema (edges well-defined) |
| 3 | Moderate to severe erythema | 3 | Moderate edema (raised approximately 1 mm) |
| 4 | Severe erythema (beet redness) and slight eschar formation | 4 | Severe edema (extending beyond area of exposure) |

Results

ACE was inhibited 100% for 24 hours and not less than 70% for 72 hours. Thus, compound (I) was absorbed in a sufficient amount and over a long time period. There was no problem in terms of skin irritation.

Example 2

[Preparation of a transdermal therapeutic composition]

In 40 g of distilled water is dispersed 4 g of compound (I) followed by addition of 0.48 g of anhydrous sodium carbonate with heating (80-100 °C) to dissolve compound (I). The solution is cooled to room temperature and 10 g of polyvinyl alcohol (completely saponified; degree of polymerization: Ca. 2,000) is added and dissolved with heating and stirring. Thereafter, 20 g of glycerol is added and after stirring, 3 g of Tween 80 and 3 g of lauryl alcohol are added and stirred to homogenize. Finally the emulsion is uniformly diluted with distilled water to make 100 g and cast onto a silicone rubber sheet provided with a recess measuring 3 $cm^2$ x 1 mm. The resulting preparation is frozen and, then, thawed to give a polyvinyl alcohol gel-type transdermal therapeutic composition.

[Transdermal absorption test]

The test procedure described in Example 1 was followed.

[Dermal irritation test]

The test procedure described in Example 1 was followed.

Results

ACE was inhibited 100% for 24 hours and not less than 70% for 72 hours. Thus, compound (I) was absorbed in a sufficient amount and over a long time. Moreover, there was no skin irritation problem, with the exception of very slight erythema.

Example 3

[Preparation of a transdermal therapeutic composition]

In 40 g of distilled water is dispersed 4 g of compound (I) followed by addition of 0.48 g of anhydrous sodium carbonate and the mixture is heated (at 80 - 100 °C) to dissolve compound (I). This solution is cooled to room temperature and, then, 8 g of polyvinyl alcohol (completely saponified; degree of polymerization: Ca. 2,000) is added and dissolved with heating and stirring. Thereafter, 20 g of 1,3-butanediol is added and, after mixing, 1 g of HCO-60, 5 g of lauric acid and 10 g of squalane are added with stirring to give an emulsion. Finally the emulsion is diluted with distilled water to make 100 g, stirred well to homogenize, and cast onto a silicone rubber sheet provided with a recess measuring 3 $cm^2$ x 1 mm . The resulting preparation is frozen and, then, thawed to give a polyvinyl alcohol gel-type transdermal therapeutic composition.

[Transdermal absorption test]

The test procedure described in Example 1 was followed.

[Dermal irritation test]

The test procedure described in Example 1 was followed.

Results

ACE was inhibited 100% for 48 hours and not less than 70% for 96 hours. Thus, compound (I) was absorbed in a sufficient amount and over a long time. Moreover, there was no skin irritation problem, with the exception of the slight erythema noted.

Reference Example

[Preparation of a transdermal therapeutic composition]

In 28 g of distilled water is dispersed 4 g of compound (I) followed by addition of 0.48 g of anhydrous sodium carbonate and the mixture is heated (at 80 - 100 °C) to dissolve compound (I). This solution is cooled to room temperature and, then, 5 g of polyvinyl alcohol (completely saponified; degree of polymerization: ca. 2,000) is added and dissolved with heating and stirring. Thereafter, 60 g of propylene glycol is added and mixed. Finally the emulsion is diluted with distilled water to make 100 g, stirred well to

homogenize, and cast onto a silicone rubber sheet provided with a recess measuring 3 $cm^2$ x 1 mm. The preparation is frozen and, then, thawed to give a polyvinyl alcohol gel-type transdermal therapeutic composition.

[Transdermal absorption test]

The test procedure described in Example 1 was followed.

[Dermal irritation test]

The test procedure described in Example 1 was followed.

Results

ACE was inhibited only 10% for 10 hours and compound (I) was hardly absorbed. Moreover, moderate erythema and slight edema were observed.

## Claims

1. A transdermal therapeutic composition which contains:
(i) (R)-3-[(S)-1-carboxy-5-(4-piperidyl)pentyl]amino-4-oxo-2,3,4,5-tetrahydro-1,5-benzothiazepine-5-acetic acid;
(ii) an inorganic base;
(iii) at least one member selected from the group consisting of an aliphatic carboxylic acid containing 6 to 20 carbon atoms, a lower alcohol ester of an aliphatic carboxylic acid containing 6 to 20 carbon atoms, and an aliphatic alcohol containing 6 to 20 carbon atoms; and
(iv) an alkanepolyol.

2. A transdermal therapeutic composition according to claim 1, wherein the inorganic base is an alkali metal hydroxide, an alkali metal carbonate or an alkali metal hydrogen carbonate.

3. A transdermal therapeutic composition according to claim 1 or 2, wherein the component (iii) is a lower alcohol ester of an aliphatic carboxylic acid containing 6 to 20 carbon atoms.

4. A transdermal therapeutic composition according to any one of claims 1 to 3, wherein the alkanepolyol is an alkanediol containing 2 to 5 carbon atoms or an alkanetriol containing 2 to 5 carbon atoms.

5. A transdermal therapeutic composition according to any one of claims 1 to 4, which contains a nonionic surfactant in addition to said components (i), (ii), (iii) and (iv).

6. A transdermal therapeutic composition according to any one of claims 1 to 5, which contains:
(i) (R)-3-[(S)-1-carboxy-5-(4-piperidyl)pentyl]amino-4-oxo-2,3,4,5-tetrahydro-1,5-benzothiazepine-5-acetic acid in a proportion of 0.1 to 20% (w/w);
(ii) an inorganic base in a proportion of 0.02 to 5% (w/w);
(iii) at least one member selected from the group consisting of an aliphatic carboxylic acid containing 6 to 20 carbon atoms in a proportion of 0.5 to 10% (w/w), a lower alcohol ester of an aliphatic carboxylic acid containing 6 to 20 carbon atoms in a proportion of 1 to 50% (w/w), and an aliphatic alcohol containing 6 to 20 carbon atoms in a proportion of 0.5 to 10% (w/w);
(iv) an alkanepolyol in a proportion of 1 to 30% (w/w); and
(v) a nonionic surfactant in a proportion of 0.1 to 10% (w/w).

7. A transdermal therapeutic composition according to any one of claims 1 to 6, which is in a dosage form of patch, cataplasma, ointment, hard ointment, tape, suppository, lotion, solution, suspension, emulsion or aerosol.

8. A transdermal therapeutic formulation characterized in that it comprises a composition as defined in any one of claims 1 to 6 and a solvent, a suspending agent, an emulsifier, a propellant,an ointment base or a suppository.

9. A transdermal therapeutic agent containing at least a composition as defined in any one of claims 1 to 6 which is absorbed in or adhered to an appropriate support material.

10. A transdermal therapeutic composition, formulation or agent, as defined in any one of claims 1 to 9 for its use for the prophylactic or therapeutic treatment of hypertension.

European Patent Office

# EUROPEAN SEARCH REPORT

Application Number

EP 89 11 5687

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 156 455 (TAKEDA)<br>* Claims 1,16,18,23,27-28; page 103: "Preparation Example" *<br>--- | 1-10 | A 61 K 31/55<br>A 61 K 47/00<br>A 61 L 15/16 |
| A | CHEMICAL ABSTRACTS, vol. 109, 1988, page 16, abstract no. 85717f, Columbus, Ohio, US; Y. INADA et al.: "(R)-3-[(S)-1-carboxy-5-(4-piperidinyl)pentyl] amino-4-oxo-2,3,4,5-tetrahydro-1,5-benzothiazepine-5-acetic acid (CV-5975): a new potent and long-lasting inhibitor of angiotensin converting enzyme", & JPN. J. PHARMACOL. 1988, 47(2), 135-41<br>* Abstract *<br>----- | 1-10 | |
| | | | TECHNICAL FIELDS SEARCHED (Int. Cl.5) |
| | | | A 61 K<br>A 61 L |

The present search report has been drawn up for all claims

| Place of search | Date of completion of the search | Examiner |
|---|---|---|
| THE HAGUE | 09-11-1989 | SCARPONI U. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document